# NEW EUROPEAN PATENT SPECIFICATION

(11) **EP 1 476 758 B2**
(45) Date of publication and mention of the opposition decision: **11.02.2015**
(45) Mention of the grant of the patent: 09.09.2009
(21) Application number: 03709934.8
(22) Date of filing: 14.02.2003
(51) Int. Cl.: G01N 33/68

(54) **DETECTION AND/OR MONITORING OF SYNUCLEIN-RELATED DISEASES**
DETEKTION UND/ODER BEOBACHTUNG VON SYNUCLEIN-ASSOZIIERTEN KRANKHEITEN
DETECTION ET/OU SURVEILLANCE DE MALADIES LIEES A LA SYNUCLEINE

(30) Priority: 14.02.2002 GB 0203446
(43) Date of publication of application: 17.11.2004
(73) Proprietor: Lancaster University Business Enterprises Limited, Lancaster LA1 4YW (GB)
(72) Inventor: ALLSOP, David, Carnforth, Lancashire LA6 1NF (GB); EL-AGNAF, Omar M.A., Morecambe, Lancashire LA3 3SE (GB); SALEM, Sultan A., Lancaster, Lancashire LA1 1SY (GB)
(74) Representative: Atkinson, Peter Birch
(86) International application number: PCT/GB2003/000677
(87) International publication number: WO 2003/069332

(56) References cited:
- WO-A-00/02053
- WO-A-99/50300
- KIM S H ET AL: "Characterization of alpha-synuclein in human lymphocytes" SOCIETY FOR NEUROSCIENCE ABSTRACTS, vol. 27, no. 1, 2001, page 862 XP001155386 31st Annual Meeting of the Society for Neuroscience;San Diego, California, USA; November 10-15, 2001 ISSN: 0190-5295
- JIA TONGLI ET AL: "Stimulation of breast cancer invasion and metastasis by synuclein gamma" CANCER RESEARCH, vol. 59, no. 3, 1 February 1999 (1999-02-01), pages 742-747, XP002258023 ISSN: 0008-5472
- EL-AGNAF OMAR M A ET AL: "Alpha-synuclein implicated in Parkinson's disease is present in extracellular biological fluids, including human plasma." THE FASEB JOURNAL: OFFICIAL PUBLICATION OF THE FEDERATION OF AMERICAN SOCIETIES FOR EXPERIMENTAL BIOLOGY. UNITED STATES OCT 2003, vol. 17, no. 13, October 2003 (2003-10), pages 1945-1947, XP002258024 ISSN: 1530-6860

## Description

The present invention relates to the detection and/or monitoring of those diseases associated with abnormalities (e.g. in the level and/or nature of the protein expressed) in the proteins called 'the synucleins'.

The synucleins are a family of small proteins (∼14 kDa) that are expressed at high levels in nervous tissue. The three members of the family (α-, β-, and γ-synuclein) are the products of three genes present on different chromosomes (1). Diseases associated with abnormalities in one or more of the synucleins are referred to here as the 'synucleinopathies'.

The synucleinopathies include some important neurodegenerative conditions, for example Parkinson's disease (PD), dementia with Lewy bodies (DLB), Alzheimer's disease (AD) and multiple system atrophy (MSA). The synucleins are also expressed at abnormally high levels in various tumours (e.g. breast, ovarian) in human cancer (2).

The first indication of an involvement of the α-synuclein member of this family in the pathogenesis of neurodegenerative diseases came from the isolation of one of its proteolytic fragments from purified amyloid of Alzheimer's diseased (AD) brains (3). This α-synuclein fragment, representing about 10% of the sodium dodecyl sulphate (SDS) insoluble material, was named non-Aβ-compouent of AD amyloid (NAC). Amino acid sequencing revealed that NAC comprised at least 35 amino acids, although the N- terminal residues could not be assigned with certainty because of the specificity of the enzyme used in sequencing. These 35 amino acids were later shown to correspond to residues 61-95 of a 140 amino-acid precursor (NACP). NACP was found to be identical with the protein called α-synuclein. A clear link with PD was established when it was shown that two different mutation in the α-synuclein gene were found in rare inherited forms of this disease. One mutation, α-synuclein(A53T), has been found in certain Italian and Greek families, and results in an Ala⁵³ to Thr substitution (4). The other mutation, α-synuclein(A30P), has been found in a family of German origin, and results in an Ala³⁰ to Pro change (5).

Lesions in the brain know as 'Lewy bodies' and 'Lewy neurites' constitute the main pathological features in the brains of patients with PD and DLB. These Lewy bodies and Lewy neurites contain the α-synuclein in an unusual fibrillar form (6). Additional immunohistochemical and immunoelectron microcopy studies have shown that α-synuclein is also associated with pathological lesions in other neurodegenerative diseases, sometimes involving non-neuronal cells, such as the glial cytoplasmic inclusions found in MSA (7). These and other neurodegenerative diseases involving α-synuclein are collectively known as the 'synucleinopathies'. In PD and DLB, α-and β-synuclein have been detected in presynaptic axon terminals, whereas γ-synuclein has been detected in abnormal axonal spheroid-like lesions (7).

Despite the progress, which has been made in understanding the underling disease mechanism of the synucleinopathies, there remains an urgent need to develop methods for use in the diagnosis of these diseases. The need for such diagnostic methods is amply demonstrated by studies indicating that a correct diagnosis of PD during life is surprisingly difficult, being correct only about 75% of the time (8, 9). This is a serious problem, not only from a clinical standpoint, but also because it affects the integrity of clinical trials and epidemiological research.

On the basis of the evidence outlined above the detection of synucleins is a good marker for diagnosing these conditions. In patients suffering from neurodegeneration it is believed that synuclein proteins are retained within the brain. As a result techniques have been proposed to analyse cerebrospinal fluid (CSF) for the presence or otherwise of synucleins in order to provide a diagnosis of a neurodegenerative disorder.

Thus, for example, WO-A-00/02053 (Innogenetics N.V.) specifically discloses detection of α-synuclein in CSF for the purposes of differential diagnosis of neurodegenerative conditions. At the more general level WO-A-00/02053 discloses detection of a wide range of selected markers of neurodegeneration (including, but not only, α-synuclein) in body fluids such as blood, lymph, urine and CSF. There is no specific disclosure in WO-A-00/02053 of detecting α-synuclein or, for that matter, any other synuclein in anything other than CSF, thus supporting the current thinking to which reference is made above.

There are, however, a number of disadvantages associated with the analysis of CSF for the presence of a synuclein for the purposes of detecting a neurodegenerative condition. In particular techniques for the collection of CSF are technically demanding of those taking the sample, and frequently a source of discomfort for those providing the sample.

Similar disadvantages also exist if it is desired to monitor the effectiveness of a drug which has been administered. Such monitoring may, for example, be effected to assess the effectiveness of the drug in the treatment of a given patient, or alternatively in an experimental model to assess the suitability of drugs for the treatment of synucleinopathies.

It is an object of the present invention to overcome the disadvantages associated with the prior art.

According to a first aspect of the present invention there is provided a method of diagnosing Parkinson's Discase in a patient comprising analysing a serum or plasma sample from the patient for the amount in said sample of an aggregated α-synuclein and comparing said amount with a reference value.

Diagnosis of a Parkinson's Disease may be performed based upon the results of the analysis.

According to a second aspect of the present invention there is provided a method of monitoring the effectiveness of a therapeutic agent that has been administered to an animal for the purpose of treating Parkinson's Disease, the method comprising analysing a serum or plasma sample taken from the animal for the amount in said sample of an aggregated α-synuclein and comparing said amount with a reference value.

The effectiveness of a therapeutic agent may be monitored using the results of the analysis undertaken. An effective therapeutic agent may be one that causes a decrease in the amount of aggregated α-synuclein present in the sample taken, as compared to a reference value. The reference value may reflect the amount of aggregated α-synuclein in a sample taken from the same patient before administration of the therapeutic agent, or may be representative of a typical amount of aggregated α-synuclein to be found in samples from entreated patients, or may represent the amount of aggregated α-synuclein to be found in a sample taken from an animal model that has received an ineffective agent. The "typical" amount of aggregated α-synuclein to be found may be determined by analysis of samples taken from appropriate control groups.

The present invention is based upon the surprising finding that, contrary to current thinking, aggregated α-synucleins are present in blood and serum from patients with Parkinson's Disease. It will be appreciated that the collection of these samples for analysis in accordance with the invention is considerably more convenient than the need to analyse CSF samples as proposed in the prior art.

The presence of aggregated α-synuclein, or of aggregated α-synuclein at a level higher than a "background" level found in all patients, may be used to distinguish between samples from patients suffering from Parkinson's Disease, and samples from otherwise healthy patients. It will be appreciated that diagnosis or monitoring may also be effected based on an elevated degree of aggregation of α-synuclein in a sample, and/or an elevated proportion of α-synuclein aggregated in a sample.

Methods of the invention may be conducted by determining the amount of the aggregated α-synuclein in the sample being assayed and comparing that amount with reference values which have been previously determined from samples taken from healthy individuals (i.e. not having a Parkinson's Disease) and samples taken from individuals having Parkinson's Disease at known stages of disease progression. Levels of the aggregated forms of aggregated α-synuclein will be elevated in the blood, plasma or serum from a patient with Parkinson's Disease as compared to blood, plasma or serum from a healthy individual.

The method of the first aspect of the invention may be used to effect diagnosis for a number of different purposes. For example, the method may be applied to the diagnosis of a patient to determine whether or not the patient has Parkinson's Disease. Alternatively this method may be applied to the diagnosis of a patient to determine the progression or extent of Parkinson's Disease.

Similarly the method of the second aspect of the invention may also be used for a number of different purposes, e.g. the monitoring of the effectiveness of an administered drug for the treatment of a patient (human or otherwise) having Parkinson's Disease. An alternative use of the method of the second aspect of the invention lies in monitoring the effectiveness of an administered drug as a potential treatment for Parkinson's Disease in an experimental model or screen, e.g. an experimental animal model or human clinical trial.

Methods of the invention may be effected by any one of a number of detection methods. The detection method may for example be a radioimmunoassay, an enzyme-linked immunosorbant assay (ELISA), a sandwich immunoassay, a fluorescent immunoassay, a precipitin reaction, a gel immunodiffusion assay, an agglutination assay, a protein A immunoassay, an immunoelectrophoresis assay, an electro phoresis and Western blotting assay, or any other technique able to measure the amount of synuclein proteins and/or their related derivatives in the sample to be tested.

Methods may, for example, involve treatment of the sample with synuclein-binding species capable of binding aggregated α-synuclein, the species being bound to a solid phase. Such a solid phase-bound species can be used to perform a pre-concentration step in which aggregated α-synuclein present within a relatively large volume of the original sample may be bound to the species. The solid phase may then be separated from the sample and treated to release the aggregated α-synuclein into a relatively smaller volume of liquid for analysis.

The solid phase to which the antibody is bound may, for instance, be a magnetic bead.

In a preferred embodiment of the invention the synuclein-binding species may be an antibody capable of binding specifically to the aggregated α-synuclein. Examples of antibodies suitable for use according to the invention include the following antibodies:
Antibody 211: recognises amino acid residues 121 to 125 of α-synuclein;
Antibody N-19: recognises an N-terminal region of α-synuclein;
Antibody FL-140: raised against full length α-synuclein;
Antibody C20: forms available recognising C-terminal regions of α, β and γ-synuclein
(all from Santa Cruz Biotechnology); and
Antibody LB509: recognises amino acid residues 115 to 122 of α-synuclein
(available from Zymed Laboratories).

Alternatively the aggregated α-synuclein-binding species may, for example, be a protein fragment capable of binding specifically to the aggregated α-synuclein, or an antibody to another molecule which is part of a complex with aggregated α-synuclein.

The presence of the aggregated α-synuclein (e.g. in the product of the preconcentration step) may be detected by performing an immuno-blotting technique using an antibody reactive to the aggregated α-synuclein.

In another embodiment of the invention the presence of the aggregated α-synuclein within either the original sample or the product of the preconcentration step may be detected by performing an enzyme linked immuno-sorbent assay (ELISA). The aggregated form of α-synuclein can be detected using a single antibody recognizing an epitope present only once within the aggregated α-synuclein as both the capture and detection antibodies.

The identity of substances detected by aggregated α-synuclein assays may further be investigated by tandem mass spectroscopy of protein eluted from, for example, gels or blots, or alternatively by analysis of N-terminal amino acid sequence data.

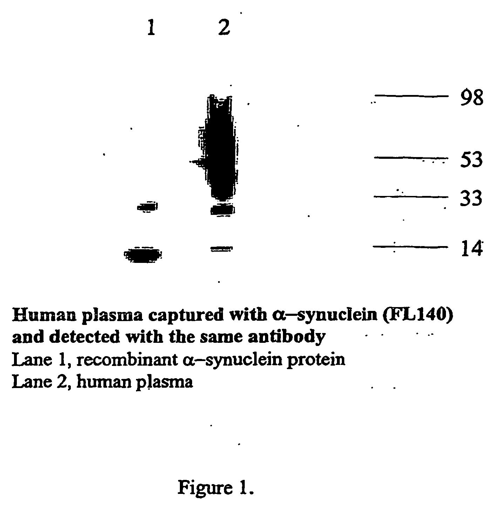

The invention will be further described by way of example only with reference to the following non-limiting examples, protocols and Figures 1 to 4 of the accompanying drawings which show the results of Examples 1 and 2.

### EXAMPLE 1 (Reference)

### Detection of α-, β-, and γ-synuclein proteins in human plasma and CSF by immunoprecipitation, gel electrophoresis and western blotting

### a) Dynabeads:

Magnetic polystyrene beads coated with recombinant protein A (Dynal Biotech) were derivatized with the capture antibody, according to the manufacturer's instructions. The antibody used for capturing the synucleins from plasma or CSF was usually α/β/γ-synuclein (FL-140) from Santa Cruz Biotechnology, but other antibodies to α-, β-, or γ-synuclein can be used for the same purpose. If the beads were to be used several times, then the capture antibodies were covalently crosslinked to the dynabeads according to the instructions of the manufacturer. Before use, the beads were extensively washed with 0.1 M phosphate buffer (pH 8.1) in 1.5m1 plastic tubes, with the beads being collected onto the wall of the tubes following each wash by placing the tubes in a Dynal Magnetic Partial Concentrator (Dynal MPC).

### b) Immunoprecipition:

The CSF or plasma sample, containing α-, β-, and γ-synuclein proteins (1ml), was added directly to the antibody-coated beads and agitated overnight at 4°C. After extensive washing with 0.1 M phosphate buffer (pH 8.1) the immunoprecipited material was eluted from the beads by boiling them in 4X NUPAGE LDS polyacrylamide gel electrophoresis sample buffer (Invitrogen).

### c) Gel Electrophoresis:

The elutate from the beads was fractionated by electrophoresis on neutral pH SDS-NUPAGE 4-12% Bis-Tris acrylamide gels (from Invitrogen). Protein markers of known molecular weights were used to calibrate the gels.

### d) Western Blotting:

After running the gels, the separated proteins were transferrer onto nitrocellulose membranes using the Invitrogen Xcell II Blot Module (at 200V, 125mA, for I hr).

After transfer, the membrane was washed extensively with PBS/Tween 20 (PBST), then blocked with blocking buffer (PBS containing 5% dried milk powder) for 1hr at room temperature. Various different antibodies were used to detect the α-, β-, and γ- synuclein proteins, or their modified forms (e.g. nitrated or phosphorylated forms), on the blots. Examples of suitable antibodies include α-synuclein (C-20), β-synuclein (C-20) and γ-synuclein (C-20), α-, β- and γ-synuclein (FL-140), all from Santa Cruz Biotechnology, or antibodies to nitrotyrosine or phosphoserine (Sigma). The membrane was incubated with primary antibody overnight at 4°C, then washed extensively with PBS/Tween 20, prior to incubation with a secondary horseradish peroxidase-conjugated antibody (1hr at room temperature) directed at the immunoglobulin species of the primary antibody. The membrane was washed extensively with PBST and peroxidase activity was detected by enhanced chemoluminescence (ECL).

### e) Results:

Figure 1 illustrates the results of Western blotting analysis of a human plasma sample in which preconcentration has been performed using an antibody reactive to α-synuclein (FL140) and detection performed with the same antibody. Lane 1 contains recombinant α-synuclein protein, and lane 2 contains human plasma.

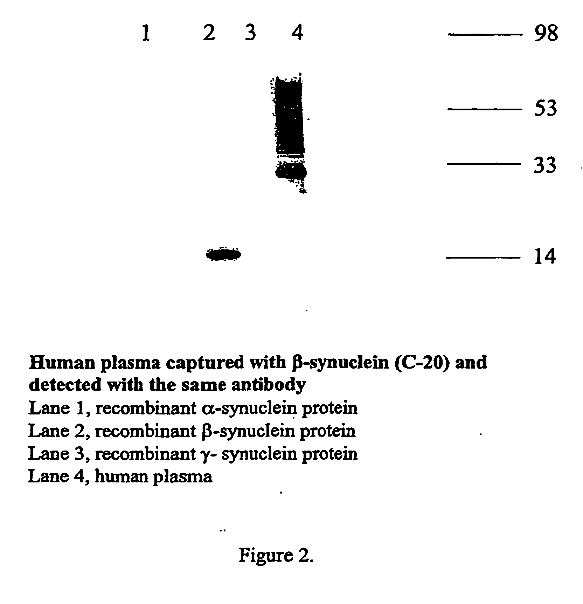

Figure 2 illustrates the results of Western blotting analysis of a human plasma sample in which preconcentration has been performer using an antibody reactive to α-synuclein (FL140) and detection performed with the same antibody. Lance 1 contains recombinant α-synuclein protein, and lane 2 contains human plasma.

Figure 2 illustrates the results of Western blotting analysis of a human plasma sample in which preconcentration has been performed using an antibody reactive to β-synuclein (C20) and detection performed with the same antibody. Lane 1 contains recombinant α-synuclein protein, lane 2 contains recombinant β-synuclein protein, lane 3 contains recombinant γ-synuclein protein and lane 4 contains human plasma.

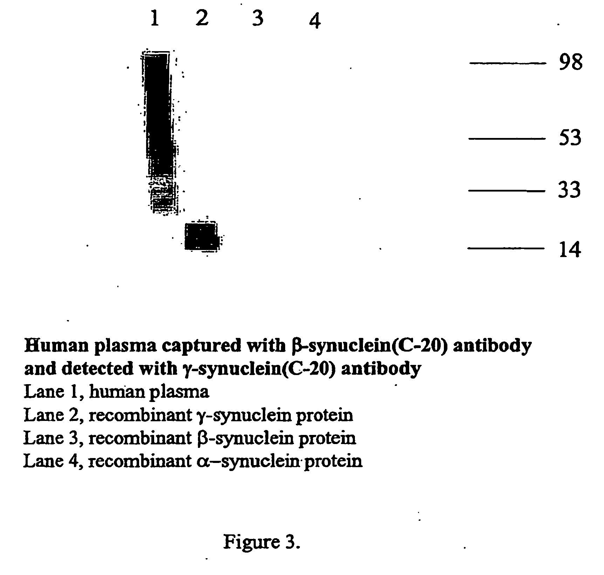

Figure 3 illustrates the results of Western blotting analysis of a human plasma sample in which preconcentration has been performed using an antibody reactive to β-synuclein (C20) and detection performed with an antibody to γ-synuclein (C20). Lane 1 contains human plasma, lane 2 contains recombinant γ-synuclein protein, lane 3 contains recombinant β-synuclein protein and lane 4 contains recombinant α-synuclein protein.

### EXAMPLE 2 (Reference)

### Detection of α-synuclein in plasma from patients with synucleinopathies.

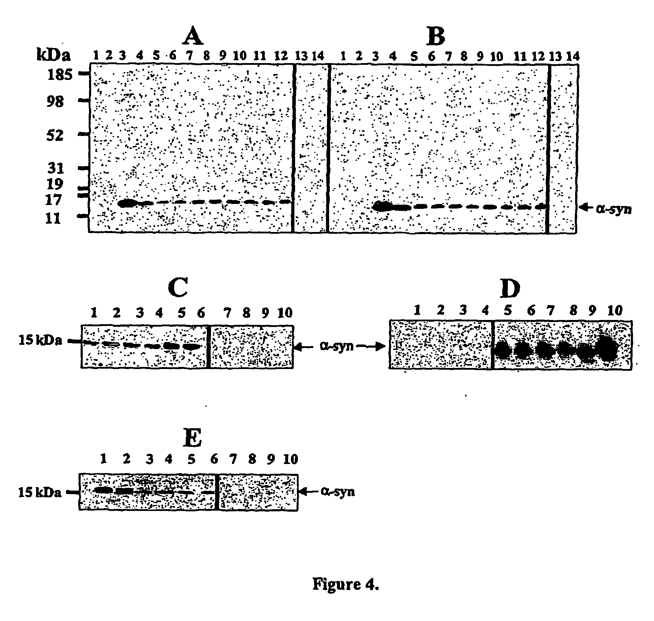

The ability to detect synucleins in plasma, including plasma of human patients suffering from synucleinopathies, was demonstrated in the following experiments, the results of which are illustrated in Figure 4 (Panels A to E).

Plasma from patients suffering from synucleinopathies (Parkinson's disease and dementia with Lewy bodies) and from age-matched control patients was incubate with magnetic beads cross-linked to antibodies reactive to α-synuclein (listed below) according to the method described in Example 1, in order to achieve a pre-concentration step.

The resultant immunoprecipitates were fractionated by SDS-PAGE, and western blotting performed (according to the methods described above), using the antibodies set out below. The detergent SDS breaks down any aggregates of synuclein that may be present in samples investigated in this manner, and so the results described below do not provide information as to the presence, or otherwise, of aggregated synuclein in the samples tested, but rather serve to illustrate the ability of the methods described to identify synuclein present in non-cerebral body fluid samples.

The gels from which the Western blots were taken comprised at least one lane containing α-synuclein positive control material, chosen from exogenous recombinant human α-synuclein and/or normal human brain lysates (since α-synuclein is found at high levels in brain tissue).

In the cases of Examples 2.1 and 2.2 the gels additionally comprised lanes containing exogenous recombinant human β- and γ-synuclein. The lack of labelling of protein from these lanes illustrates that the antibodies used for Western blotting are able to specifically bind α-synuclein as opposed to other synuclein isoforms.

### 2.1 Immunoprecipitation with antibody FL-140; Western blotting with antibody LB509.

Immunoprecipitation was carried out using primary antibody FL-140. Western blotting was performed using antibody LB509. The results are shown in the photograph of the Western blot membrane in Figure 4A, for which:

| | |
|---|---|
| *Lane 1:* | 6ng recombinant human β-synuclein. |
| *Lane 2:* | 6ng recombinant human γ-synuclein. |
| *Lane 3:* | 6ng recombinant human α-synuclein. |
| *Lane 4:* | 3µg normal human brain lysates. |
| *Lanes 5, 6, 13:* | Plasma from patients with Parkinson's disease. |
| *Lanes 7, 8:* | Plasma from patients with DLB. |
| *Lanes 9-12, 14:* | Plasma from age-matched control patients. |

Lanes 13 and 14 were probed with LB509 that had been pre-adsorbed with recombinant human α-synuclein (1µg α-synuclein/ml antibody). The lack of binding seen in these two lanes illustrates the specificity for α-synuclein of the LB509 antibody.

The results illustrate the ability of the assay to detect endogenous synuclein in plasma samples from both patients with synucleinopathies and control patients, and also in positive controls (exogenous α-synuclein and brain lysates).

### 2.2 Immunoprecipitation with antibody FL-140; Western blotting with antibody 211.

Example 2.1 was repeated, save that immunoprecipitation was carried out using primary antibody FL-140 and Western blotting was performed using antibody 211. The results are shown in Figure 2B

Lanes 13 and 14 were probed with antibody 211 that had been pre-adsorbed with recombinant human α-synuclein (1µg α-synuclein/ml antibody). The lack of binding seen in these two lanes illustrates the specificity for α-synuclein of the 211 antibody.

The results illustrate the ability of the assay to detect endogenous synuclein in control and synucleinopathy patient plasma samples, as well as in positive controls.

### 2.3 Immunoprecipitation with antibody FL-140; Western blotting with antibody N-19.

Immunoprecipitation was carried out using primary antibody FL-140. Western blotting was performed using antibody N-19. The results are shown in Figure 4C, and the samples investigated were as set out below:

| | |
|---|---|
| Lanes 1, 2, 7: | Plasma from patients with Parkinson's disease. |
| Lane 3, 4, 8, 9: | Plasma from age-matched control patients. |
| Lane 5: | 3 µg normal hum an brain lysates. |
| Lanes 6, 10: | 6ng recombinant human α-synuclein. |

Lanes 7 to 10 were probed with antibody N-19 that had been pre-adsorbed with recombinant human α-synuclein (1µg α-synuclein/ml antibody). The lack of binding seen in these two lanes illustrates the specificity for α-synuclein of the N-19 antibody.

The results illustrate the ability of the assay to detect endogenous synuclein in plasma samples and also in positive controls.

### 2.4 Immunoprecipitation with antibody 211; Western blotting with antibody FL-140.

Immunoprecipitation was carried out using primary antibody FL-140. Western blotting was performed using antibody 211. The results are shown in Figure 4D, and the samples investigated were as set out below:

| | |
|---|---|
| Lanes 1, 5, 6: | Plasma from patients with Parkinson's disease. |
| Lanes 2, 3, 7, 8: | Plasma from age-matched control patients. |
| Lanse 4, 9: | 6ng recombinant human α-synuclein. |
| Lane 10: | 3µg normal human brain lysates. |

Lanes 1 to 4 were probed with antibody FL-140 that had been pre-adsorbed with recombinant human α-synuclein (1µg α-synuclein/ml antibody). The lack of binding seen in these two lanes illustrates the specificity for α-synuclein of the FL-140 antibody.

The results illustrate the ability of the assay to detect endogenous synuclein in patient plasma samples, and also to detect synuclein in positive controls.

### 2.5 Immunoprecipitation with antibody 211; Western blotting with antibody N-19.

Immunoprecipitation was carried out using primary antibody 211, and Western blotting performed using antibody N-19. The results are shown in Figure 4E, and the samples investigated were as set out below:

| | |
|---|---|
| Lanes 1, 10: | 6ng recombinant human α-synuclein. |
| Lane 2: | 3µg normal human brain lysates. |
| Lanes 3, 4, 7: | Plasma from patients with Parkinson's disease. |
| Lanes 5, 6, 8, 9: | Plasma from age-matched control patients. |

Lanes 7 to 10 were probed with antibody N-19 that had been pre-adsorbed with recomabinant human α-synuclein (1µg α-synuclein/ml antibody). The lack of binding seen in these two lanes illustrates the specificity for α-synuclein of the N-29 antibody.

The results illustrate the ability of the assay to detect endogenous synuclein in patient plasma samples, and also to detect synuclein in positive controls.

### EXAMPLE 3 (Reference)

### Measuring soluble α-Synuclein protein in human plasma by ELISA

### A. REAGENTS

### 1. Coating buffer

200 mM NaHCO3 (Sigma) pH 9.6. The solution is then filtered through a 0.2 µm filter (Aldrich).

### 2. Blocking solution

The blocking solution consists of 100 ml of PBS buffer (pH 7.4) containing 2.5% gelatin (Sigma) and 0.05% Tween 20 (Sigma). This solution should be made fresh and used in the same day and does not require filtering.

### 3. Biotinylation of antibodies

Antibodies were biotinylated as recommended by the biotin manufacturer "PIERCE".

### B. METHOD

1. Coat plate (Nunc, maxisorp) with 100 µl/well of the α-synuclein (C-20) antibody affinity-purified goat polyclonal antibody raised against the C-terminus of α-synuclein (Santa Cruz Biotechnology, Inc. Cat. No SC-7011) 1:1000 (2 µg/ml) in 200 mM NaHCO3 (pH 9.6). The plate is then covered with a plate sealer (Sigma) and cling-film and stored overnight at 4°C.
2. Aspirate N-19 antibody from the plate by inverting over the sink. Wash 4 times with PBS-Tween 20 (0.05%) (PBST).
3. Block the plate by using 200 µl/well blocking buffer. Incubate the plate at 37°C for 1 hr.
4. Wash the plates 4 times with PBST (PBS buffer containing 0.05% Tween-20).
5. Add 100 µl/well of plasma or serum. Cover the plate and incubate for 2 hrs at 37°C.
6. Wash the plate 4 times with PBST. Dilute the biotinylated N-19 antibody affinity-purified goat polyclonal antibody raised against the N-terminus of α-synuclein (Santa Cruz Biotechnology, Inc. Cat. No sc-7012) at a dilution 1:1000 (0.2 µg/ml) in blocking buffer and add 100µl /well. Incubate for 2 hrs at 37°C.
7. Wash 4 times with PBST.
8. Add 100 µl/well of ExtrAvidin-Alkaline phosphatase (Sigma) diluted 3:5000 in blocking buffer and incubate for 1 hr at 37°C.
9. Wash the plate 4 times in PBST. Add 100 µl/well of Alkaline phosphatase Yellow "pNPP" and incubate for 30 min at RT. Read absorbance values at 405 nm.

### C. RESULTS

Using the above procedure, human plasma, collected from healthy individuals not suffering from a synucleinopathy, was found to contain α-synuclein at a concentration greater than 1µM.

### Example 4

### Measurement and comparison of amount of aggregated synuclein in plasma of Parkinson's disease patients and control patients.

Plasma samples from n=34 patients with clinically diagnosed Parkinson's disease and n=27 age-matched control patients were analysed by ELISA performed as described below in Protocol 2 for measuring multimeric (aggregated) α-synuclein. The ELISA protocol selected is a sandwich ELISA, and is specific for aggregated (oligomerised) forms of synuclein.

An increase in absorbance at 405nm is indicative of increased presence of aggregated synuclein in the sample tested. Absorbance was measured in optical density units (OD).

Plasma samples from the clinically diagnosed Parkinson's disease patients had mean absorbance of 1.7 OD +/- standard deviation (SD) of 0.7057. In contrast the plasma samples from age-matched control patients had mean absorbance of 0.236 OD +/- SD of 0.396. These values are strikingly different, with a much higher mean value in the group of patients suffering from the synucleinopathy Parkinson's disease.

The difference in absorbance, and hence levels of aggregated synuclein, between groups was shown to be significant on statistical analysis.

A Fisher's exact probability test, conducted with an absorbance value of 0.5 OD assigned to represent a "high" level of oligomerised synuclein, gave a highly significant difference between the values obtained for the Parkinson's disease and control groups (P = 0.002). Selection of a cut-off point of 0.25 OD absorbance for Fisher's exact probability test produced an even more significant difference (P = 0.001). Thus, absorbance values above either of these two selected cut-off points are highly indicative of the presence of Parkinson's disease.

### PROTOCOLS

### Protocol 1 for Measuring multimeric (aggregated) α-Synuclein protein

### A. REAGENTS

### 1. Coating buffer

200 mM NaHCO3 (Sigma) pH 9.6. The solution is then filtered through a 0.2 µm filter (Aldrich).

### 2. Blocking solution

The blocking solution consists of 100 ml of PBS buffer (pH 7.4) containing 2.5% gelatin (Sigma) and 0.05% Tween 20 (Sigma). This solution should be made fresh and used in the same day and does not require filtering.

### 3. Biotinylation of antibodies

Antibodies were biotinylated as recommended by the biotin manufacturer "PIERCE".

### B. METHOD

1. Coat plate (Nunc, maxisorp) with 100 µl/well, of the N-19 antibody affinity-purified goat polyclonal antibody raised against the N-terminus of α-synuclein (Santa Cruz Biotechnology, Inc. Cat. No sc-7012) at a dilution 1:1000 (0.2 µg/ml) in 200 mM NaHCO3 (pH 9.6). The plate is then covered with a plate sealer (Sigma) and cling-film and stored overnight at 4°C.
2. Aspirate N-19 antibody from the plate by inverting over the sink. Wash 4 times with PBS-Tween 20 (0.05%) (PBST).
3. Block the plate by using 200 µl /well blocking buffer. Incubate the plate at 37°C for 1 hr.
4. Wash the plates 4 times with PBST.
5. Add 100 µl/well of plasma or serum. Cover the plate and incubate for 2 hrs at 37°C.
6. Wash the plate 4 times with PBST. Dilute the biotinylated N-19 antibody 1:2000 (1 µg/ml) in blocking buffer and add 100µl /well. Incubate for 2 hrs at 37°C.
7. Wash 4 times with PBST.
8. Add 100 µl/well of ExtrAvidin-Alkaline phosphatase (Sigma) diluted 3:5000 in blocking buffer and incubate for 1 hr at 37°C.
9. Wash the plate 4 times in PBST. Add 100 µl /well of Alkaline phosphatase Yellow "pNPP" and incubate for 30 min at RT. Read absorbance values at 405 nm.

### Protocol 2 for Measuring multimeric (aggregated) α-Synuclein protein

### A. REAGENTS

### 1. Coating buffer

200 mM NaHCO3 (Sigma) pH 9.6. The solution is then filtered through a 0.2 µm filter (Aldrich).

### 2. Blocking solution

The blocking solution consists of 100 ml of PBS buffer (pH 7.4) containing 2.5% gelatin (Sigma) and 0.05% Tween 20 (Sigma). This solution should be made fresh and used in the same day and does not require filtering.

### 3. Biotinylation of antibodies

Antibodies were biotinylated as recommended by the biotin manufacturer "PIERCE".

### B. METHOD

1. Coat plate (Nunc, maxisorp) with 100 µl/well of the α-synuclein (211) (mouse monoclonal IgG antibody which recognises amino acid 121-125 of α-synuclein (Santa Cruz Biotechnology, Inc. Cat. No sc-12767) 1:100 (2 µg/ml) in 200 mM NaHCO3 (pH 9.6). The plate is then covered with a plate sealer (Sigma) and cling-film and stored overnight at 4°C.
2. Aspirate 211 antibody from the plate by inverting over the sink. Wash 4 times with PBS-Tween 20 (0.05%) (PBST).
3. Block the plate by using 200 µl/well blocking buffer. Incubate the plate at 37°C for 1 hr.
4. Wash the plates 4 times with PHST.
5. Add 100 µl/well of plasma or serum. Cover the plate and incubate for 2 hrs at 37°C.
6. Wash the plate 4 times with PBST. Dilute the biotinylated α-synuclein (211) 1:200 (1 µg/ml) in blocking buffer and add 100µl /well. Incubate for 2 hrs at 37°C.
7. Wash 4 times with PBST.
8. Add 100 µl/well of ExtrAvidin-Alkaline phosphatase (Sigma) diluted 3:5000 in blocking buffer and incubate for 1 hr at 37°C.
9. Wash the plate 4 times in PBST. Add 100 µl /well of Alkaline phosphatase Yellow "pNPP" and incubate for 30 min at RT. Read absorbance values at 405 nm.

### REFERENCES.

1. Glayton, D.F. and George, J.M. (1999) Synucleins in synaptic plasticity and neurodegenerative disorders. J. Neurosci. Res. 58, 120-129.
2. Bruening, W., Giasson, B.I., Klein-Szanto, A.J., Lee, V.M., Trojanowski, J.Q. and Godwin, A.K. (2000) Synucleins are expressed in the majority of breast and ovarian carcinomas and in preneoplastic lesions of the ovary. Cancer 88, 2154-2163.
3. Ueda, K., Fukushima, H., Masliah, E., Xia, Y., Iwai, A., Otero, D., Kondo, J., Ihara, Y. and Saitoh, T. (1993) Molecular cloning of cDNA encoding an unrecognised component of amyloid in Alzheimer disease. Proc. Natl. Acad. Sci. USA 90, 11282-11286.
4. Polymeropoulos, M.H., Lavedan, C., Leroy, E., Ide, S.E., Dehejia, A., Dutra, A., Pike, B., Root, H., Rubenstein, J., Boyer, R., Stenroos, E.S., Chandrasekharappa, S., Athanassiadou, A., Papapetropoulos, T., Johnson, W.G., Lazzarini, A.M., Duvoisin, R.C., Di lorio, G., Golbe, L.I. and Nussbaum, R.L. (1997) Mutation in the α-synuclein gene identified in families with Parkinson's disease. Science 276, 2045-2047.
5. Kruger, R., Kuhn, W., Muller, T., Woitalla, D., Graeber, M., Kosel, S., Przuntek, H., Epplen, J.T., Schols, L. and Riess, O. (1998) Ala30Pro mutation in the gene encoding α-synuelein in Parkinson's disease. Nature Gen. 18, 106-108.
6. Spillantini, M.G., Crowther, R.A., Jakes, R., Cairns, N.J., Lantos, P.L. and Goedert, M. (1998) Filamentous alpha-synuclein inclusions link multiple system atrophy with Parkinson's disease and dementia with Lewy bodies. Neurosci Lett. 251 ,205-208.
7. Galvin, J.E., Uryu, K., Lee, V.M. and Trojanowski, J.Q. (1999) Axon pathology in Parkinson's disease and Lewy body dementia hippocampus contains alpha-, beta-, and gamma-synuclein. Proc. Natl. Acad. Sci. USA 96,13450-13455.
8. Hushes, A.J., Ben-Shlomo, Y., Daniel, S.E. and Lees, A.J. (1992) What features improve the accuracy of clinical diagnosis in Parkinson's disease. Neurology 42, 1142-1146.
9. Rajput, A.H., Rozdilsky, B., and Rajput, A. (1991) Accuracy of clinical diagnosis in Parkinson's disease-a prospective study. Can. J. Neurol. Sci. 18, 275-278.
10. Shimura, H., Schlossmacher, M.G., Hattori, N., Frosch, M.P., Trockenbacher, A., Schneider, R., Mizuno, Y., Kosik, K.S., Selkoe, D.J. (2001) Ubiquitination of a new form of α-synuclein by Parkin from human brain: Implications for Parkinson's disease. Science, 293, 263-269.

## Claims

1. A method of diagnosing Parkinson's Disease in a patient comprising analysing a serum or plasma sample from the patient for the amount in said sample of an aggregated α-synuclein and comparing said amount with a reference value.

2. A method of monitoring the effectiveness of a therapeutic agent that has been administered to an animal for the purpose of treating Parkinson's Disease, the method comprising analysing a serum or plasma sample taken from the animal for the amount in said sample of an aggregated α-synuclein and comparing said amount with a reference value.

3. A method according to claim 1 or claim 2, wherein the sample is plasma.

4. A method according to any preceding claim, wherein the analysis for the presence of aggregated α-synuclein is conducted by a method selected from a radioimmunoassay, an enzyme-linked immunosorbant assay (ELISA), a sandwich immunoassay, a fluorescent immunoassay, a precipitin reaction, a gel immunodiffusion assay, an agglutination assay, a protein A immunoassay, an immunoelectrophoresis assay and an electrophoresis and Western blotting assay.

5. A method according to any preceding claim, comprising:
i) treatment of the sample with a synuclein-binding species capable of binding aggregated α-synuclein, or capable of binding any molecule which is part of a complex with aggregated α-synuclein, said species being bound to a solid phase, to perform a pre-concentration step;
ii) removal of the solid phase from the sample; and optionally
iii) treatment of the solid phase to release the synuclein into a relatively smaller volume of liquid for analysis.

6. A method according to claim 5, wherein the solid phase to which the species is bound is a magnetic bead or other suitable matrix.

7. A method according to claim 1 wherein the analysis is effected by treatment of the sample with a synuclein binding species capable of binding aggregated α-synuclein, the species being bound to a solid phase.

8. A method according to claim 9 wherein the synuclein binding species is an antibody capable of binding the aggregated α-synuclein.

## Patentansprüche

1. Verfahren zur Diagnose der Parkinson-Krankheit bei einem Patienten, umfassend das Analysieren einer Serum- oder Blutplasmaprobe von dem Patienten zum Bestimmen der in der Probe enthaltenen Menge eines aggregierten α-Synucleins und das Vergleichen der Menge mit einem Bezugswert.

2. Verfahren zum Beobachten der Wirksamkeit eines Therapeutikums, das einem Tier zum Zweck der Behandlung der Parkinson-Krankheit verabreicht worden ist, wobei das Verfahren umfasst: Analysieren einer von dem Tier entnommenen Serum- oder Blutplasmaprobe zum Bestimmen der in der Probe enthaltenen Menge eines aggregierten α-Synucleins und Vergleichen der Menge mit einem Bezugswert.

3. Verfahren nach Anspruch 1 oder Anspruch 2, wobei die Probe Blutplasma ist.

4. Verfahren nach einem der vorstehenden Ansprüche, wobei die Analyse zur Ermittlung der Gegenwart von aggregiertem α-Synuclein nach einem Verfahren durchgeführt wird, das ausgewählt ist aus einem Radioimmuntest, einem Enzymimmuntest (ELISA), einem Sandwich-Immuntest, einem Fluoreszenz-Immuntest, eine Präzipitinreaktion, einem Gel-Immunodiffusionstest, einem Agglutinationstest, einem Protein A-Immuntest, einem Immunoelektrophoresetest und einem Elektrophorese- und Western-Blotting-Test.

5. Verfahren nach einem der vorstehenden Ansprüche, umfassend:
i) Behandlung der Probe mit einer synuclein-bindenden Spezies, die imstande ist, aggregiertes α-Synuclein zu binden oder irgendein Molekül zu binden, das Teil eines Komplexes mit aggregiertem α-Synuclein ist, wobei die Spezies an eine feste Phase gebunden ist, um einen Vorkonzentrationsschritt durchzuführen;
ii) Entfernen der festen Phase aus der Probe; und wahlweise
iii) Behandlung der festen Phase zur Freisetzung des Synucleins in ein relativ kleineres Flüssigkeitsvolumen zur Analyse.

6. Verfahren nach Anspruch 5, wobei die feste Phase, an welche die Spezies gebunden ist, eine Magnetperlen- oder andere geeignete Matrix ist.

7. Verfahren nach Anspruch 1, wobei die Analyse ausgeführt wird, indem die Probe mit einer synuclein-bindenden Spezies behandelt wird, die aggregiertes α-Synuclein binden kann, wobei die Spezies an eine feste Phase gebunden ist.

8. Verfahren nach Anspruch 9, wobei die synuclein-bindende Spezies ein Antikörper ist, der das aggregierte α-Synuclein binden kann.

## Revendications

1. Procédé de diagnostic d'une maladie de Parkinson chez un patient, comprenant l'analyse d'un échantillon de sérum ou de plasma du patient quant à la quantité, dans ledit échantillon, d'une α-synucléine agrégée, et la comparaison de ladite quantité avec une valeur de référence.

2. Procédé de suivi de l'efficacité d'un agent thérapeutique qui a été administré à un animal afin de traiter une maladie de Parkinson, le procédé comprenant l'analyse d'un échantillon de sérum ou de plasma prélevé de l'animal quant à la quantité, dans ledit échantillon, d'une α-synucléine agrégée, et la comparaison de ladite quantité avec une valeur de référence.

3. Procédé selon la revendication 1 ou la revendication 2, dans lequel l'échantillon est du plasma.

4. Procédé selon l'une quelconque des revendications précédentes, dans lequel l'analyse quant à la présence d'α-synucléine agrégée est effectuée par un procédé choisi parmi un radioimmunoessai, un dosage immunoenzymatique (ELISA), un immunoessai en sandwich, un immunoessai fluorescent, une réaction de précipitation, un essai d'immunodiffusion sur gel, un essai d'agglutination, un immunoessai de protéine A, un essai d'immunoélectrophorèse et un essai d'électrophorèse et de transfert Western.

5. Procédé selon l'une quelconque des revendications précédentes, comprenant:
i) le traitement de l'échantillon avec une espèce se liant à une α-synucléine, apte à se lier à une α-synucléine agrégée, ou apte à se lier à toute molécule qui fait partie d'un complexe avec une α-synucléine agrégée, ladite espèce étant liée à une phase solide, pour effectuer une étape de préconcentration;
ii) l'enlèvement de la phase solide à partir de l'échantillon; et facultativement
iii) le traitement de la phase solide pour libérer la synucléine dans un volume de liquide relativement inférieur pour l'analyse.

6. Procédé selon la revendication 5, dans lequel la phase solide à laquelle l'espèce est liée est une bille magnétique ou une autre matrice appropriée.

7. Procédé selon la revendication 1, dans lequel l'analyse est effectuée par traitement de l'échantillon avec une espèce se liant à une α-synucléine, apte à se lier à une α-synucléine agrégée, l'espèce étant liée à une phase solide.

8. Procédé selon la revendication 9, dans lequel l'espèce se liant à une α-synucléine est un anticorps apte à se lier à l'α-synucléine agrégée.
